# FASCICULE DE BREVET EUROPEEN

(11) **EP 3 828 763 B1**
(45) Date de publication et mention de la délivrance du brevet: **14.08.2024**
(21) Numéro de dépôt: 20210468.3
(22) Date de dépôt: 27.11.2020
(51) Int. Cl.: G06V 40/13, G06V 40/20, A61B 5/00, A61B 5/053, G01B 11/24, A61B 5/1172, G06V 40/12

(54) **CAPTEUR D'EMPREINTE À DÉTECTION D'IMPÉDANCE**
IMPEDANZERKENNUNG FINGERDRUCKSENSOR
IMPEDANCE DETECTION FINGERPRINT SENSOR

(30) Priorité: 29.11.2019 FR 1913508
(43) Date de publication de la demande: 02.06.2021
(73) Titulaire: Idemia Identity & Security France, 92400 Courbevoie (FR)
(72) Inventeur: MAILLARD, Sylvain, 92400 COURBEVOIE (FR); CHARTIER, Anne, 92400 COURBEVOIE (FR); SANDRAZ, Jean-Rémi, 92400 COURBEVOIE (FR); MORICEAU, Aurélie, 92400 COURBEVOIE (FR)
(74) Mandataire: Idemia

(56) Documents cités:
- EP-A2- 0 786 745
- WO-A1-2015/130809
- WO-A1-2018/155346
- WO-A1-97/14111
- TOSHISHIGE SHIMAMURA ET AL: "Impedance-Sensing Circuit Techniques for Integration of a Fraud Detection Function Into a Capacitive Fingerprint Sensor", IEEE SENSORS JOURNAL., vol. 12, no. 5, 1 May 2012 (2012-05-01), US, pages 1393 - 1401, XP055500685, ISSN: 1530-437X, DOI: 10.1109/JSEN.2011.2172413

## Description

La présente invention concerne le domaine de la biométrie et plus particulièrement le domaine des capteurs d'empreinte.

Il est connu des systèmes de reconnaissance biométrique permettant l'identification d'individu par la reconnaissance d'un dermatoglyphe et plus particulièrement d'une empreinte digitale.

Un tel système comprend un dispositif de détection d'empreinte comprenant un élément sensible associé à un support du doigt de manière que l'élément sensible puisse capturer une image du dermatoglyphe lorsqu'un doigt est appliqué contre le support. L'élément sensible est très souvent un capteur optique mais d'autres technologies existent comme la détection capacitive, la détection de champ électrique, la détection thermique...

Des fraudeurs ont tenté de tromper les systèmes de reconnaissance biométrique à partir d'empreintes en utilisant un faux doigt sur lequel a été réalisé un dermatoglyphe d'une autre personne. Ces faux doigts sont dans des matériaux ayant des propriétés différentes de celles de la peau et du corps humain en général.

Pour démasquer ces fraudeurs, la surface du support en contact avec le doigt a été pourvue d'électrodes reliées par des pistes à un circuit de détection d'impédance entre les électrodes : l'impédance entre électrodes (égale à la somme de l'impédance de contact entre chacune des électrodes et la peau du doigt et de l'impédance à l'intérieur du doigt) est différente dans un faux doigt que dans un vrai.

Dans un mode de réalisation classique, le support est en verre et les pistes et électrodes sont formées par un dépôt de matériau électriquement conducteur à la surface du verre. Il se trouve que les pistes sont fragiles et que l'interruption d'une piste, par exemple par une rayure, entraîne une augmentation importante de l'impédance faisant croire de façon erronée à un faux doigt.

Les pistes sont recouvertes d'une couche d'isolant électrique assurant également une relative protection des pistes contre une abrasion résultant du contact avec le doigt.

Cependant, il arrive que la couche de protection soit altérée par exemple du fait de frottements répétés lors d'une utilisation intensive et/ou lors d'opérations de nettoyage qui sont d'autant plus fréquentes que l'utilisation du dispositif de détection est intensive. Cette altération peut également conduire à l'interruption d'une des pistes conductrices. WO97/14111A1 et WO2015/130809A1 proposent des capteurs d'empreinte ayant des électrodes et deux pistes conductrices s'étendant sur un support entre les électrodes et un circuit électronique de mesure d'impédance.

### OBJET DE L'INVENTION

L'invention a notamment pour but d'améliorer la fiabilité des dispositifs de détection d'empreintes.

### RESUME DE L'INVENTION

A cet effet, on prévoit, selon l'invention définie dans la revendication 1 un dispositif de détection d'empreinte, comprenant un support, un capteur agencé pour capturer une image d'un dermatoglyphe d'un doigt d'utilisateur posé sur le support, et un circuit électronique de mesure d'impédance relié à des électrodes s'étendant sur le support, des pistes conductrices s'étendant sur le support entre les électrodes et le circuit électronique de mesure d'impédance. Au moins deux des électrodes sont reliées chacune au circuit électronique de mesure d'impédance par au moins deux pistes conductrices, dans lequel les pistes conductrices reliées à une même électrode sont inclinées l'une par rapport à l'autre.

Ainsi, même si l'une des pistes conductrices reliant l'électrode au circuit électronique de mesure d'impédance est coupée en particulier par abrasion ou rayure, l'électrode reste reliée au circuit électronique de mesure d'impédance par l'autre piste conductrices. Le risque que toutes les pistes reliant une électrode au circuit de mesure d'impédance soient coupées est limité.

D'autres caractéristiques et avantages de l'invention ressortiront à la lecture de la description qui suit d'un mode de réalisation particulier et non limitatif de l'invention.

### BREVE DESCRIPTION DES DESSINS

Il sera fait référence aux dessins annexés, parmi lesquels :
La figure 1 est une vue schématique d'un dispositif de détection d'empreinte selon l'invention ;
La figure 2 est une vue schématique d'un agencement d'électrodes selon un premier mode de réalisation ;
La figure 3 est une vue schématique d'un agencement d'électrodes selon un deuxième mode de réalisation.

### DESCRIPTION DETAILLEE DE L'INVENTION

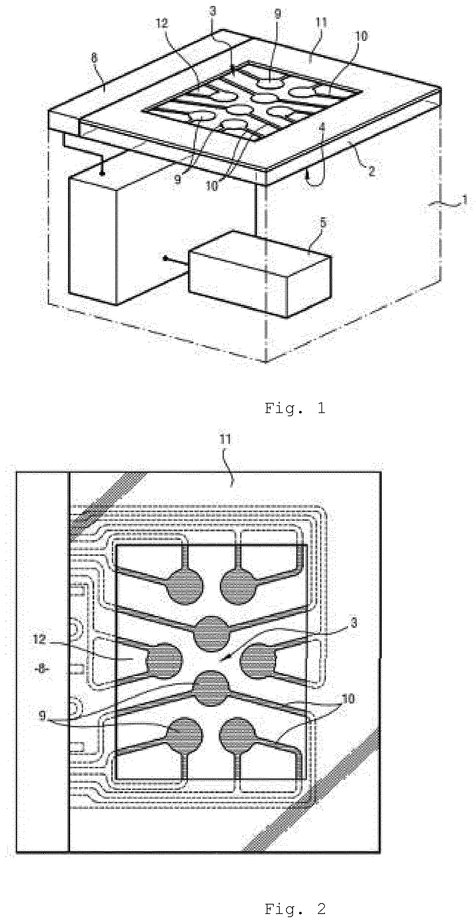

En référence aux figures 1 et 2, le dispositif de détection d'empreinte selon l'invention comprend un boîtier 1 portant un support 2 ici sous la forme d'une lame de verre ou tout autre matériau transparent. Le support 2 ayant une surface externe 3 (tournée vers l'extérieur du boîtier 1) formant une surface d'appui pour un doigt (le support fournit une résistance mécanique empêchant que le doigt ne se déplace dans la direction selon laquelle il est appliqué contre la surface externe 3) et une surface interne 4 (tournée vers l'intérieur du boîtier 1) en regard de laquelle est montée un capteur optique 5 relié à une unité électronique de commande 6 enfermée avec le capteur optique 5 dans le boîtier 1. Le capteur optique 5, connu en lui-même, est par exemple un capteur de type CCD ou CMOS et est éventuellement disposé derrière un groupe optique comprenant au moins une lentille. L'unité électronique de commande 6 comprend de manière connue en elle-même un processeur et une mémoire pour exécuter un programme comprenant des instructions agencées de manière connue en elle-même pour :
- commander le capteur optique 5 en vue d'acquérir une image de la surface d'un doigt posée sur la surface externe 3,
- extraire des caractéristiques biométriques de l'images capturée,
- comparer les caractéristiques biométriques extraites à des caractéristiques biométriques de référence et calculer un score de similarité reflétant le résultat de cette comparaison,
- comparer le score de similarité à un seuil déterminé de telle manière que, lorsque le score de similarité est supérieur au seuil, la probabilité pour que les caractéristiques biométriques extraites et les caractéristiques biométriques de référence appartiennent à une même personne est de X%. Ce seuil est un compromis entre un taux souhaité de faux rejet (les caractéristiques biométriques extraites et les caractéristiques biométriques de référence sont considérées de manière erronée comme n'appartenant pas à la même personne) et un taux souhaité de fausse acceptation (les caractéristiques biométriques extraites et les caractéristiques biométriques de référence sont considérées de manière erronée comme appartenant à la même personne).

L'unité électronique de commande 6 est reliée à un connecteur de raccordement externe 7 monté sur une surface externe du boîtier 1. Le connecteur de raccordement externe 7 permet le raccordement du dispositif de détection d'empreinte à un ordinateur ou tout autre équipement susceptible d'être alimenté par les données biométriques fournies par l'unité électronique de commande 6, ou à un dispositif de condamnation d'accès à un lieu ou tout autre équipement susceptible d'être commandé par l'unité de commande électronique de commande 6 en fonction du résultat d'une comparaison de données biométriques effectuée par l'unité électronique de commande 6.

L'unité électronique de commande 6 est reliée à un circuit électronique de mesure d'impédance 8 et le programme de l'unité électronique de commande 6 est agencé pour comparer les mesures d'impédance fournies par le circuit électronique de mesure d'impédance 8 à un seuil permettant à l'unité électronique de commande 6 de distinguer un vrai doigt d'un faux doigt. Le circuit électronique de mesure d'impédance 8 est relié à des électrodes 9 via des pistes électriquement conductrices 10. Les électrodes 9 et les pistes conductrices 10 sont en un matériau électriquement conducteur déposé sur la surface externe 3 ici par un procédé de dépôt sous vide (le matériau conducteur peut initialement recouvrir la totalité de la surface puis être gravé pour former les électrodes, mais d'autres procédés sont envisageables comme un procédé d'impression au moyen d'une encre électriquement conductrice par exemple). Les électrodes 9 s'étendent dans la zone centrale 12 de la surface externe 3. Les pistes conductrices 10 reliant des électrodes 9 différentes sont écartées d'une distance suffisante pour limiter le risque de couplage électrique ou électromagnétique entre lesdites pistes conductrices 10.

La surface externe 3 est recouverte d'une couche électriquement isolante 11 (représentée sur la figure 1 plus épaisse qu'elle ne l'est en réalité) qui recouvre également les pistes conductrices 10 et qui présente une ouverture de forme rectangulaire laissant découverte la zone centrale 12 de la surface externe 3 où se trouvent les électrodes 9. Cette ouverture définit une zone de capture qui se trouve dans le champ du capteur optique 5 : au moins une partie du doigt doit recouvrir cette zone pour que le capteur optique 5 puisse capturer une image du dermatoglyphe et le doigt doit être en contact avec au moins deux électrodes pour procéder à la mesure d'impédance. Le matériau électriquement isolant constituant la couche électriquement isolante 11 est ici du dioxyde de silicium. La couche électriquement isolante 11 a ici une épaisseur de 80 nm.

Pour assurer une mesure d'impédance significative, le nombre d'électrodes 9 est déterminé pour qu'au moins quatre électrodes 9 soient en contact avec le doigt de telle manière qu'une mesure d'impédance puisse être effectuée pour six couples d'électrodes 9. De préférence, le nombre d'électrodes 9 est supérieur à quatre : lors de la capture d'une image de l'empreinte, l'unité électronique de commande 6 détecte celles des électrodes 9 qui sont en contact avec le doigt compte tenu de la position du doigt dans l'image et compare l'impédance mesurée entre lesdites électrodes en contact avec le doigt à un seuil pour déterminer si le doigt est faux ou réel (en effet, il ne faut pas tenir compte d'une impédance mesurée avec une électrode qui n'est pas en contact avec le doigt car toute impédance qui serait mesurée avec cette électrode serait très élevée et représentative de manière erronée d'un faux doigt).

Selon l'invention, chacune des électrodes 9 est reliée au circuit électronique de mesure d'impédance 8 par au moins deux pistes conductrices 10.

En référence plus particulièrement à la figure 2, selon le premier mode de réalisation de l'invention, chaque électrode 9 a une forme sensiblement circulaire, avec ici un diamètre de 4 mm environ, et est reliée au circuit électronique de mesure d'impédance 8 par deux pistes conductrices 10.

Les électrodes 9 sont réparties dans la zone centrale 12 avec des électrodes 9 s'étendant au voisinage des limites de la zone centrale 12 et des électrodes s'étendant au voisinage du milieu de la zone centrale 12.

Pour chaque électrode 9 située au voisinage d'une des limites de la zone centrale 12, les pistes conductrices 10 sont reliées à l'électrode 9 du côté de ladite limite. On limite ainsi la longueur de piste laissée découverte par la couche électriquement isolante 11.

Pour chaque électrode 9 située au voisinage du milieu de la zone centrale 12, les pistes conductrices 10 sont reliées à l'électrode 9 en des points diamétralement opposés l'un à l'autre. Ces pistes ont une longueur, laissée découverte par la couche électriquement isolante 11, qui est relativement importante : en éloignant l'une de l'autre les deux pistes, on limite le risque qu'une seule rayure n'interrompe les deux pistes.

Les pistes conductrices 10 reliées à une même électrode 9 sont inclinées l'une par rapport à l'autre, c'est-à-dire qu'elles ne sont pas parallèles l'une à l'autre.

On comprend que le risque d'interruption d'une piste conductrice par une rayure est plus important lorsque la rayure s'étend selon la plus petite dimension de la piste conductrice, soit dans sa largeur. Les deux pistes reliées à une même électrode ayant des directions différentes, une même rayure rectiligne (comme elles le sont le plus souvent) ne peut pas s'étendre dans la largeur des deux pistes conductrices. En outre, les frottements lors des opérations de nettoyage sont le plus souvent réalisés selon une même direction, de tels frottements ne risquent pas de causer des rayures s'étendant dans la largeur des deux pistes conductrices reliées à une même électrode.

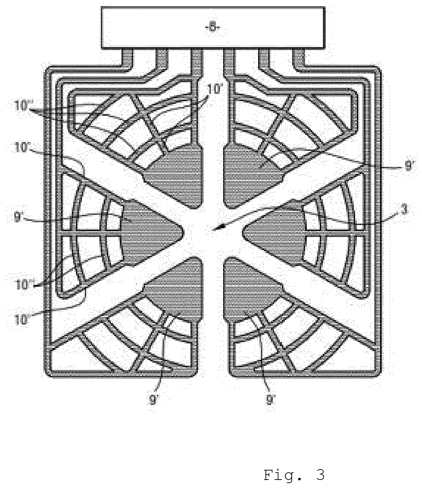

Les éléments identiques ou analogues à ceux précédemment décrits porteront la même référence numérique que ces derniers dans la description qui suit du deuxième mode de réalisation en relation avec la figure 3.

Le deuxième mode de réalisation est identique au premier sauf en ce qui concerne l'agencement des électrodes et des pistes conductrices.

Selon le deuxième mode de réalisation, les électrodes 9' s'étendant dans la zone centrale 12 de la surface externe 3 ont une forme sensiblement triangulaire avec un sommet orienté vers le milieu de la zone centrale 12.

Chaque électrodes 9' est relié au circuit électronique de mesure d'impédance par trois pistes conductrices principales 10' s'étendant entre ladite électrodes 9' et le circuit électronique de mesure d'impédance. Les pistes conductrices 10' sont de préférence reliées à un côté du triangle formé par l'électrode 9', ce côté étant celui opposé au sommet orienté vers le centre la zone centrale 12. Les pistes conductrices 10' reliées à une même électrode 9' forment entre elles un angle aigu.

Des pistes conductrices secondaires 10" relient deux à deux les pistes conductrices principales 10' entre elles de manière à relier chaque électrode 9'au circuit électronique de mesure d'impédance par un réseau de pistes conductrices 10', 10" interconnectées. Il y a ici au moins deux pistes conductrices secondaires 10" pour chaque paire de pistes conductrices principales 10'. On comprend qu'une piste conductrice principale 10', interrompue à un endroit, peut néanmoins continuer à transmettre un courant électrique sur sa partie non interrompue jusqu'à une piste conductrice secondaire 10".

Cet agencement en réseau des pistes conductrices 10', 10" rend plus robuste aux rayures le raccordement des électrodes 9 au circuit électronique de mesure d'impédance 8.

Bien entendu, l'invention n'est pas limitée aux modes de réalisation décrits mais englobe toute variante entrant dans le champ de l'invention telle que définie par les revendications.

En particulier, le dispositif de détection peut avoir une structure différente de celle décrite ou représenté.

Le nombre, la position et l'orientation des pistes conductrices principales et/ou secondaires peuvent être différents de ceux décrits ou représentés. Les pistes conductrices peuvent ainsi être parallèles les unes aux autres.

L'orientation des pistes conductrices 10 peut être différente ou identique d'une électrode à l'autre. On pourra choisir l'orientation des pistes conductrices en fonction d'une direction privilégiée de frottement, supposée ou avérée : si on s'aperçoit par exemple que les frottements sont majoritaires dans la direction de la longueur de la zone de capture, on cherchera à orienter les pistes conductrices pour que leur largeur s'étende selon une direction différente de la direction privilégiée de frottement.

Concernant le nombre de pistes, il ne faut pas que si les pistes sont en partie découvertes du fait d'une usure de la couche isolante et en contact avec un doigt, il ne faut pas qu'elles influent significativement sur l'impédance mesurée.

Le nombre, la forme, la surface et la position des électrodes peuvent être différents de ceux décrits ou représentés.

Il est possible de combiner les deux modes de réalisation décrits.

Une ou plusieurs des électrodes peuvent être reliées au circuit de mesure d'impédance par une seule piste conductrice.

La forme des pistes conductrices peut être différente de celle représentée. Avantageusement, les pistes sont arrondies pour ne pas présenter d'angles vifs notamment au niveau des intersections entre les pistes (congés de raccordement).

La forme de la zone de capture peut ne pas être rectangulaire et par exemple carrée, en croix, ronde ou ovale.

Le capteur d'empreinte peut être optique ou fonctionner selon une autre technologie.

De préférence, la couche isolante recouvre les pistes conductrices et une légère partie des électrodes pour limiter encore le risque d'interruption des électrodes. La matière de la couche isolante et/ou son épaisseur peuvent être différentes (l'épaisseur peut aller jusque 1 µm voire 2 um par exemple).

De préférence, les pistes conductrices et les électrodes s'étendent directement sur la surface externe 3 du support 2 mais, en variante, les pistes conductrices et/ou les électrodes peuvent s'étendre indirectement sur le support 2, c'est-à-dire qu'elles s'étendent sur une ou plusieurs couches intermédiaires recouvrant tout ou partie de la surface d'appui 3 du support 2.

Selon un autre exemple, un motif en oxyde d'indium-étain (ITO de l'anglais « Indium Tin Oxide ») comprenant les pistes conductrices 10, et éventuellement les électrodes 9, s'étend directement sur la surface externe 3, et un second motif en métal comprenant ces mêmes pistes conductrices 10 et éventuellement ces mêmes électrodes 9, recouvre en tout ou partie le motif en oxyde d'indium-étain pour améliorer la conduction électrique avec le doigt. Les pistes conductrices 10, et éventuellement les électrodes 9, sont avantageusement formées d'au moins une même couche conductrice, c'est-à-dire dans au moins une même couche d'un matériau conducteur.

## Revendications

1. Dispositif de détection d'empreinte, comprenant un support (2), un capteur (5) agencé pour capturer une image d'un dermatoglyphe d'un doigt d'utilisateur posé sur le support (2), et un circuit électronique de mesure d'impédance (8) relié à des électrodes (9, 9') s'étendant sur le support (2), des pistes conductrices (10, 10') s'étendant sur le support entre les électrodes (9, 9') et le circuit électronique de mesure d'impédance (8), **caractérisé en ce qu'**au moins deux des électrodes (9, 9') sont reliées chacune au circuit électronique de mesure d'impédance (8) par au moins deux pistes conductrices (10, 10'), dans lequel les pistes conductrices (10, 10') reliées à une même électrode (9, 9') sont inclinées l'une par rapport à l'autre.

2. Dispositif selon la revendication 1, dans lequel les pistes conductrices (10, 10') reliées à une même électrode (9, 9') forment entre elles un angle aigu.

3. Dispositif selon la revendication 2, dans lequel au moins une piste conductrice transversale (10'') relie entre elles les pistes conductrices (10') reliées à une même électrode (9').

4. Dispositif selon l'une quelconque des revendications précédentes, dans lequel au moins une des électrodes (9) a une forme sensiblement circulaire.

5. Dispositif selon la revendication 5, dans lequel l'électrode (9) de forme sensiblement circulaire a un diamètre d'environ 4 mm.

6. Dispositif selon l'une quelconque des revendications précédentes, dans lequel au moins une des électrodes (9') a une forme sensiblement triangulaire ayant un sommet orienté vers une zone centrale (12) de la surface d'appui (3) .

7. Dispositif selon la revendication 6, dans lequel les pistes conductrices (10') sont reliées à un côté du triangle formé par l'électrode (9'), ce côté étant celui opposé au sommet orienté vers le centre la zone centrale (12) .

8. Dispositif selon l'une quelconque des revendications précédentes, dans lequel les pistes conductrices (10) sont formées d'au moins une même couche conductrice.

## Patentansprüche

1. Vorrichtung zur Erkennung eines Abdrucks, die einen Träger (2), einen Sensor (5), der eingerichtet ist, um ein Bild eines Dermatoglyphen eines auf den Träger (2) aufgelegten Benutzerfingers aufzunehmen, und eine elektronische Impedanzmessschaltung (8) enthält, die mit Elektroden (9, 9') verbunden ist, die sich auf dem Träger (2) erstrecken, wobei Leiterbahnen (10, 10') sich auf dem Träger zwischen den Elektroden (9, 9') und der elektronischen Impedanzmessschaltung (8) erstrecken, **dadurch gekennzeichnet, dass** mindestens zwei der Elektroden (9, 9') je mit der elektronischen Impedanzmessschaltung (8) durch mindestens zwei Leiterbahnen (10, 10') verbunden sind, wobei die mit einer gleichen Elektrode (9, 9') verbundenen Leiterbahnen (10, 10') zueinander geneigt sind.

2. Vorrichtung nach Anspruch 1, wobei die mit einer gleichen Elektrode (9, 9') verbundenen Leiterbahnen (10, 10') einen spitzen Winkel zwischen sich bilden.

3. Vorrichtung nach Anspruch 2, wobei mindestens eine querliegende Leiterbahn (10") die mit einer gleichen Elektrode (9') verbundenen Leiterbahnen (10') miteinander verbindet.

4. Vorrichtung nach einem der vorhergehenden Ansprüche, wobei mindestens eine der Elektroden (9) eine im Wesentlichen kreisrunde Form hat.

5. Vorrichtung nach Anspruch 5, wobei die Elektrode (9) von im Wesentlichen kreisrunder Form einen Durchmesser von etwa 4 mm hat.

6. Vorrichtung nach einem der vorhergehenden Ansprüche, wobei mindestens eine der Elektroden (9') eine im Wesentlichen dreieckige Form hat, die einen zu einem zentralen Bereich (12) der Auflagefläche (3) gerichteten Scheitel hat.

7. Vorrichtung nach Anspruch 6, wobei die Leiterbahnen (10') mit einer Seite des von der Elektrode (9') gebildeten Dreiecks verbunden sind, wobei diese Seite die dem zur Mitte des zentralen Bereichs (12) gerichteten Scheitel gegenüberliegende ist.

8. Vorrichtung nach einem der vorhergehenden Ansprüche, wobei die Leiterbahnen (10) von mindestens einer gleichen leitenden Schicht gebildet werden.

## Claims

1. Print-detecting device, comprising a rest (2), a sensor (5) arranged to capture an image of a dermatoglyphic print of a user's finger placed on the rest (2), and an electronic impedance-measuring circuit (8) connected to electrodes (9, 9') extending over the rest (2), conductive tracks (10, 10') extending over the rest between the electrodes (9, 9') and the electronic impedance-measuring circuit (8), **characterized in that** at least two of the electrodes (9, 9') are each connected to the electronic impedance-measuring circuit (8) by at least two conductive tracks (10, 10'), the conductive tracks (10, 10') connected to a given electrode (9, 9') being inclined with respect to each other.

2. Device according to Claim 1, wherein the conductive tracks (10, 10') connected to a given electrode (9, 9') make an acute angle to each other.

3. Device according to Claim 2, wherein at least one transverse conductive track (10'') connects to each other the conductive tracks (10') connected to a given electrode (9').

4. Device according to any of the preceding claims, wherein at least one of the electrodes (9) has a substantially circular shape.

5. Device according to Claim 5, wherein the electrode (9) of a substantially circular shape has a diameter of about 4 mm.

6. Device according to any of the preceding claims, wherein at least one of the electrodes (9') has a substantially triangular shape having a vertex oriented towards a central area (12) of the contact surface (3).

7. Device according to Claim 6, wherein the conductive tracks (10') are connected to one side of the triangle formed by the electrode (9'), this side being the one opposite the vertex oriented towards the centre of the central area (12).

8. Device according to any of the preceding claims, wherein the conductive tracks (10) are formed from at least one same conductive layer.
